# EUROPEAN PATENT APPLICATION

(11) **EP 4 571 307 A1**
(43) Date of publication of application: **18.06.2025**
(21) Application number: 23854470.4
(22) Date of filing: 16.08.2023
(51) Int. Cl.: G01N 33/493, E03B 9/00

(54) **TEST DEVICE, TOILET AND TEST METHOD**

(30) Priority: 17.08.2022 CN 202210988026; 17.08.2022 CN 202222169091 U; 11.10.2022 CN 202222669648 U; 24.11.2022 CN 202223133820 U; 23.04.2023 CN 202320932034 U; 23.04.2023 CN 202320931822 U
(71) Applicant: XIAMEN R&T PLUMBING TECHNOLOGY CO., LTD., Xiamen, Fujian 361028 (CN)
(72) Inventor: HUANG, Candong, Xiamen, Fujian 361028 (CN); ZHANG, Zulian, Xiamen, Fujian 361028 (CN); LIN, Jian, Xiamen, Fujian 361028 (CN); LIN, Yunpeng, Xiamen, Fujian 361028 (CN); ZHONG, Zhijun, Xiamen, Fujian 361028 (CN)
(74) Representative: Loo, Chi Ching
(86) International application number: PCT/CN2023/113390
(87) International publication number: WO 2024/037570

(57) **Abstract**

Provided in the present invention are a test device, a toilet and a test method. The test device comprises a test module, and further comprises a container body used for storing the test module. The interior of the container body is provided with a plurality of mutually independent accommodating channels; the test module comprises a test element, and a liquid under test is tested by means of the test element; several test modules are provided and mounted in the accommodating channels respectively, and each test module can independently extend out of the corresponding accommodating channel. By mounting the test modules in the plurality of independent accommodating channels, the test modules are separated and used alone without contaminating each other, and the test device does not need to be replaced frequently and is convenient and hygienic to use.

## Description

### TECHNICAL FIELD

The present invention relates to the technical field of bathroom and urine test devices, in particular to a test device, a toilet and a test method.

### BACKGROUND

In modem society, people are paying more and more attention to health. Urine is one of the important metabolites that reflect the health status of the body, and urine test is developing from relying on specific places (hospitals) and specific personnel (medical staff) to ordinary toilet locations and being measurable by everyone. Therefore, toilets with urine test functions have emerged.

Currently, existing urine test devices on the market usually rely on urine test paper for testing, devices used for storing test elements are mostly uniformly fixed and placed, and the test elements are not separated. To avoid mutual contamination between the test elements, they need to be removed and discarded in a timely manner after each use, which is time-consuming and laborious, with poor user experience.

### SUMMARY

The objective of the present invention is to provide a test device, a toilet and a test method. By configuring a plurality of independent accommodating channels inside a container body of a storage container and mounting test modules therein respectively, each test module is separated and used alone to avoid the problems in the prior art.

In order to solve the above problems, the present invention is implemented through the following technical solutions:

A test device includes a test module, and further includes a container body used for storing the test module, where the interior of the container body is provided with a plurality of mutually independent accommodating channels; the test module includes a test element, a liquid under test is tested by means of the test element, several test modules are provided and mounted in the accommodating channels respectively, and each test module can independently extend out of the corresponding accommodating channel.

In the test device provided by the present invention, the plurality of independent accommodating channels are used for mounting the test modules respectively, such that each test module is separated and used alone without contaminating each other, multiple times of independent test can be implemented, and the test device does not need to be frequently replaced, making it convenient and hygienic to use.

Further, when the container body is not assembled onto a toilet, the test element is prone to falling from a rear cover assembly. The present invention is implemented through the following technical solution:

The test module is movable between a test position and a storage position; the accommodating channel is provided with a limit member, and the limit member is movable between a limiting position and a yielding position; when the limit member moves to the limiting position, the limit member is in limiting fit with the test module, and the test module is limited to the storage position; when the limit member moves to the yielding position, the limit member is in yielding fit with the test module, and the test module can move between the test position and the storage position.

The limit member is in limiting/yielding fit with the test assembly, such that the test module can be securely stored on the container body, which prevents the test module from falling off without interfering with the normal ejection and use of the test module.

Further, in order to ensure that the test module can fully reach a designated position and ensure accurate test results and stable operation, the present invention is implemented through the following technical solution:

The test device further includes: a bracket having a base; and a transmission member provided with a clamping portion; one end of the test module is provided with a connecting portion; when the container body drives the test module to move to a first position, the clamping portion separates from the connecting portion, such that the transmission member separates from the test module; when the container body drives the test module to move to a second position, the connecting portion is in limiting fit with the clamping portion, such that the transmission member is in transmission fit with the test module.

The container body moves and drives the test module for limiting or separation, which can easily achieve the clamping and decoupling of the transmission member and the test module and avoid easy decoupling, with a reliable and stable structure.

Further, existing storage containers for storing test paper cores are generally fixed at specific positions, with one used for storing clean unused test paper cores and the other used for collecting used and discarded test paper cores, resulting in a large product volume and large space occupation. In addition, the storage containers are inconvenient to disassemble and assemble, and therefore, are not conducive to maintenance and replacement. The present invention is implemented through the following technical solution:

A test device includes: a bracket provided with a base; and a test assembly mounted in the base, where the test assembly includes a container body and a rear cover, a plurality of test modules are mounted in the container body, and the rear cover is movable between a locking position and an unlocking position; when the rear cover moves to the locking position, the rear cover limits the container body in the base; when the rear cover moves to the unlocking position, the container body can exit from the base.

When the rear cover moves to the locking position, a fit portion is in limiting fit with a limit portion, and then the container body is limited in the accommodating chamber by the rear cover; when the rear cover moves to the unlocking position, the fit portion is in yielding fit with the limit portion, and then both the rear cover and the container body can exit the accommodating chamber from an opening. Such design can achieve fast and convenient disassembly and assembly of the test assembly and the bracket for replacement and repair, with a simple structure and strong practicality.

Further, currently the entire test assembly is mounted in the entire device, and no limit structure for limiting the test assembly is not configured in the prior art. As a result, the test assembly cannot be limited at a working position. In severe cases, the test assembly may detach from the base to affect the operation of a urine test mechanism. To solve this problem, the present invention is implemented through the following technical solution:

A test device includes: a bracket having a base; a test assembly detachably mounted in the base; a driving mechanism used for driving the test assembly to move; a positioning member movably arranged on the bracket, where when the positioning member moves to a third position, the positioning member can be in limiting fit with the test assembly; when the positioning member moves to a fourth position, the positioning member can be in yielding fit with the test assembly; a second transmission mechanism used for driving the positioning member to move; and a main control board electrically connected to the driving mechanism and the second transmission mechanism respectively to control the operation of the driving mechanism and the second transmission mechanism.

The positioning member is configured to limit the test assembly, which can ensure that the test assembly moves to the working position and prevent the test assembly from detaching from the base, with a simple structure and reliable functions.

The present invention further provides a toilet including the aforementioned test device, where a toilet bowl of the toilet is provided with a test hole into which the test element extends to test a liquid under test.

Further, in the toilet, the test device further includes a driving mechanism and a plugging element, where the driving mechanism is in transmission fit with the container body to drive the container body to move; the plugging element is arranged in one of the accommodating channels in an extensible and retractable manner, and the plugging element after extending out can be in sealing fit with the test hole; and
the position of the test hole corresponds to the test module or the plugging element; when test is performed, the driving mechanism drives the container body to move, such that the test module corresponds to the test hole; when no test is performed, the driving mechanism drives the container body to move, such that the plugging element corresponds to the test hole.

Further, the toilet further includes a first transmission mechanism; when the test module corresponds to the test hole, the first transmission mechanism drives the test element to extend out from the test hole for test; when the plugging element corresponds to the test hole, the first transmission mechanism drives the plugging element to extend out to plug the test hole.

The present invention further provides a test method, including the following process:
providing a container body for storing test modules, where the container body is provided with a plurality of mutually independent accommodating channels inside, the test module is mounted in the accommodating channel, and the test module is provided with a test element for test a liquid under test; a first transmission mechanism drives the test module to extend out of the corresponding accommodating channel; the first transmission mechanism moves or the container body moves, and where the first transmission mechanism can drive the test module to extend out of the corresponding accommodating channel.

Further, the above method further includes the following process:
when test is performed, the container body moves to a position where one test module corresponds to a test hole of a toilet, and the first transmission mechanism drives the test module to extend out of the corresponding accommodating channel; and
after the test is completed, the test module retracts into the original accommodating channel; or discarding the test module.

Optionally, the test method further includes the following process:
after the test is completed, the container body moves to the next test module corresponding to the test hole of the toilet;
when the test is performed again, the next test module extends out of the corresponding accommodating channel; and
after the test is completed again, the test module retracts into the original accommodating channel; or discarding the test module.

Optionally, the test method further includes the following process:
when the test is performed again, the container body moves to a position where the next test module corresponds to the test hole of the toilet, and another test module extends out of the corresponding accommodating channel; and
after the test is completed again, the test module retracts into the original accommodating channel; or discarding the test module.

Compared with the prior art, the technical solutions of the present invention and the beneficial effects thereof are as follows:
(1) In the test device provided by the present invention, the plurality of independent accommodating channels are used for mounting the test modules respectively, such that each test module is separated and used alone without contaminating each other, multiple times of independent test can be implemented, and the test device does not need to be frequently replaced, making it convenient and hygienic to use.
(2) When the test device provided by the present invention is used, after all the test modules are used up, the entire container body can be detached and replaced with a new container body simply and conveniently, without the need for cleaning every time, and the user will not contact the liquid under test on the test element, so the test device is clean and hygienic.
(3) In the test device provided by the present invention, the container body is movable, and the driving mechanism has a first driving assembly in transmission fit with the container body; the first transmission mechanism has a transmission member, and the container body is movable, such that when one test module in the container body corresponds to the transmission member, the transmission member can drive the test module to move. Through the fitting of the driving mechanism and the first transmission mechanism, automatic replacement of the test module can be achieved, the test device is convenient to use, and manual replacement is not required.
(4) When the liquid under test is tested using the test device provided by the present invention, the container body moves until one test module therein corresponds to the test hole, and the test module is driven by the first transmission mechanism to extend out of the corresponding accommodating channel; after the test of the liquid under test is completed, the test module retracts into the original accommodating channel; or the test module is discarded, so an additional waste recovery box is not required, which reduces product volume and saves space.
(5) In the test device provided by the present invention, a plugging element may be further provided in the accommodating channel inside the container body. After the test is completed, the test hole formed on the toilet bowl can be sealed by the plugging element, which makes the test device more hygienic and prevents the test device from long-term contact with the toilet bowl, and the real-time contact improves the use flexibility and reliability of the test device.
(6) The limit member is in limiting/yielding fit with the test assembly, such that the test module can be securely stored on the container body, which prevents the test module from detaching without interfering with the normal ejection and use of the test module.
(7) Before the container body is assembled into the base, a locking member is held in the locking position by the elastic force of an elastic member. After the container body is assembled into the base, the locking member moves from the locking position to the unlocking position under the action of the base, thereby ensuring that the test module is stably stored in the container body before the container body is accurately assembled with the base, without detaching.
(8) The container body moves and drives the test module for limiting or separation, which can easily achieve the clamping and decoupling of the transmission member and the test module and avoid easy decoupling, with a reliable and stable structure.
(9) The positioning member is configured to limit the test assembly, which can ensure that the test assembly moves to the working position and prevent the test assembly from detaching from the base, with a simple structure and reliable functions.
(10) When the rear cover moves to the locking position, the fit portion is in limiting fit with the limit portion, and then the container body is limited in the accommodating chamber by the rear cover; when the rear cover moves to the unlocking position, the fit portion is in yielding fit with the limit portion, and then both the rear cover and the container body can exit the accommodating chamber from the opening. Such design can achieve fast and convenient disassembly and assembly of the test assembly and the bracket for replacement and repair, with a simple structure and strong practicality.

### BRIEF DESCRIPTION OF THE DRAWINGS

FIG. 1 is three-dimensional schematic diagram of a test device provided in a first embodiment of the present invention;
FIG. 2 is a cross-sectional view of the test device provided in the first embodiment of the present invention in one direction;
FIG. 3 is a three-dimensional schematic diagram of a container body of the test device provided in the first embodiment of the present invention;
FIG. 4 is a three-dimensional schematic diagram of the container body of the test device provided in the first embodiment of the present invention, which is mounted with a first driving assembly;
FIG. 5 is a schematic diagram of another test device provided in the first embodiment of the present invention;
FIG. 6 is a cross-sectional view of a toilet provided in the first embodiment of the present invention in a non-test state, where a plugging element extends out;
FIG. 7 is an enlarged view of the test device in FIG. 6;
FIG. 8 is a cross-sectional view of the toilet provided in the first embodiment of the present invention at the beginning of test, where a test kit does not extend out;
FIG. 9 is an enlarged view of the test device in FIG. 8;
FIG. 10 is a cross-sectional view of the toilet provided in the first embodiment of the present invention during test, where the test kit extends out;
FIG. 11 is an enlarged view of the test device in FIG. 10;
FIG. 12 is an exploded schematic diagram of a test device provided in a second embodiment of the present invention;
FIG. 13 is a cross-sectional view of the test device provided in the second embodiment of the present invention;
FIG. 14 is a cross-sectional view of a test assembly provided in the second embodiment of the present invention;
FIG. 15 is an exploded view of a container body, a test module, and a locking member provided in the second embodiment of the present invention;
FIG. 16 is a three-dimensional structural diagram of the container body provided in the second embodiment of the present invention;
FIG. 17 is an assembly diagram of the test assembly and a base provided in the second embodiment of the present invention;
FIG. 18 is a state diagram of a convex part and an anti-falling rib provided in the second embodiment of the present invention, where a represents an abutting state; b represents a separation state;

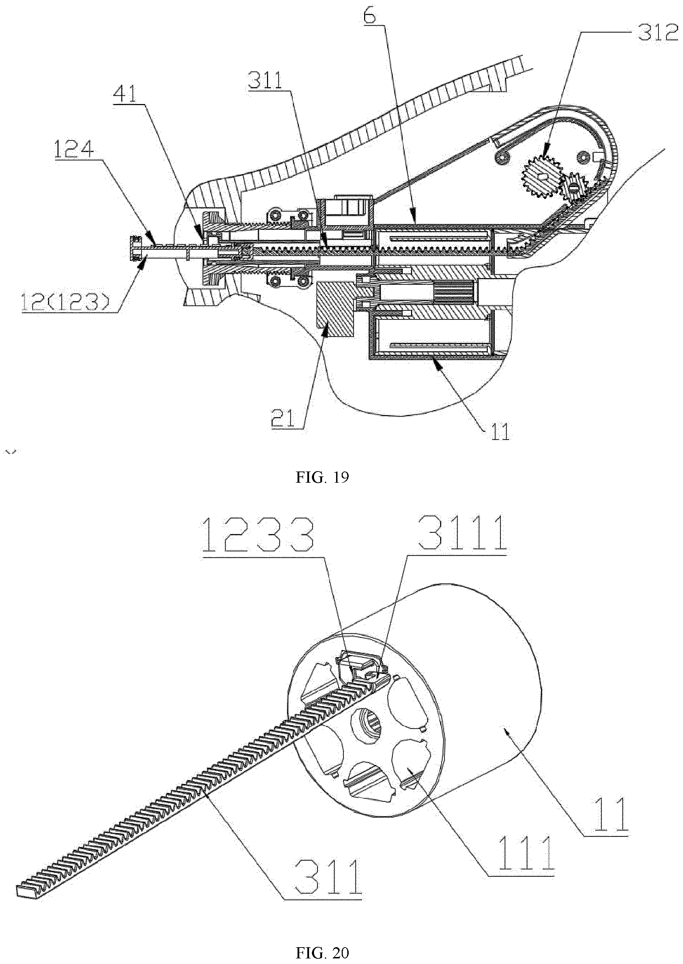

FIG. 19 is a cross-sectional view of a transmission mechanism provided in a third embodiment of the present invention;
FIG. 20 is a first assembly diagram of a container body, a test module, and a transmission member (rack) of a test device provided in the third embodiment of the present invention (separation state);

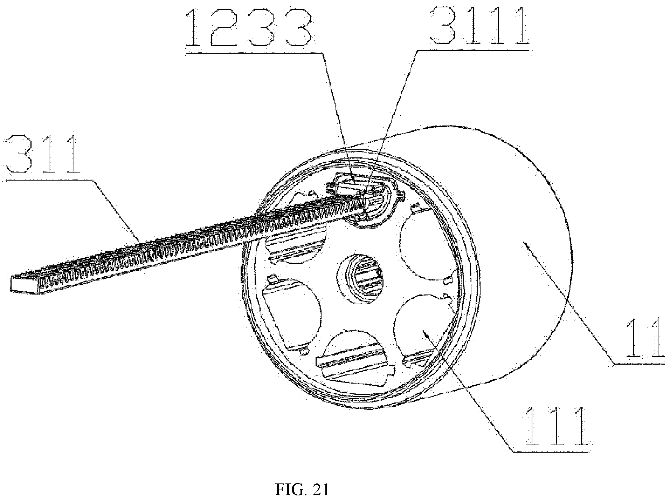

FIG. 21 is a second assembly diagram of the container body, the test module, and the transmission member (rack) of the test device provided in the third embodiment of the present invention (limiting state);

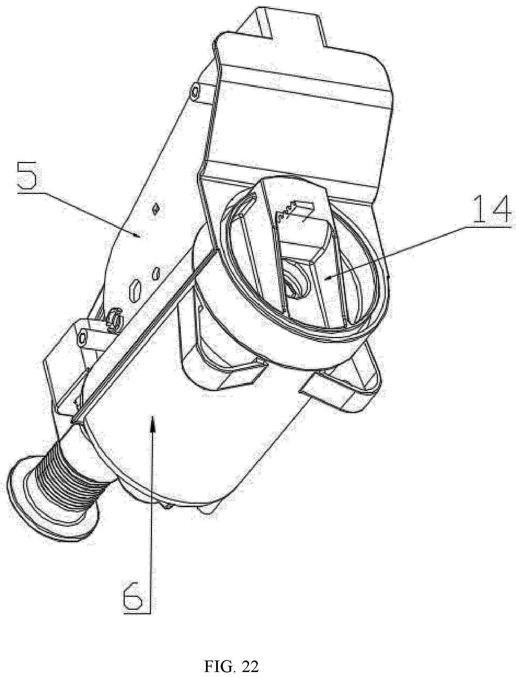

FIG. 22 is an assembly diagram of a test device provided in a fourth embodiment of the present invention;

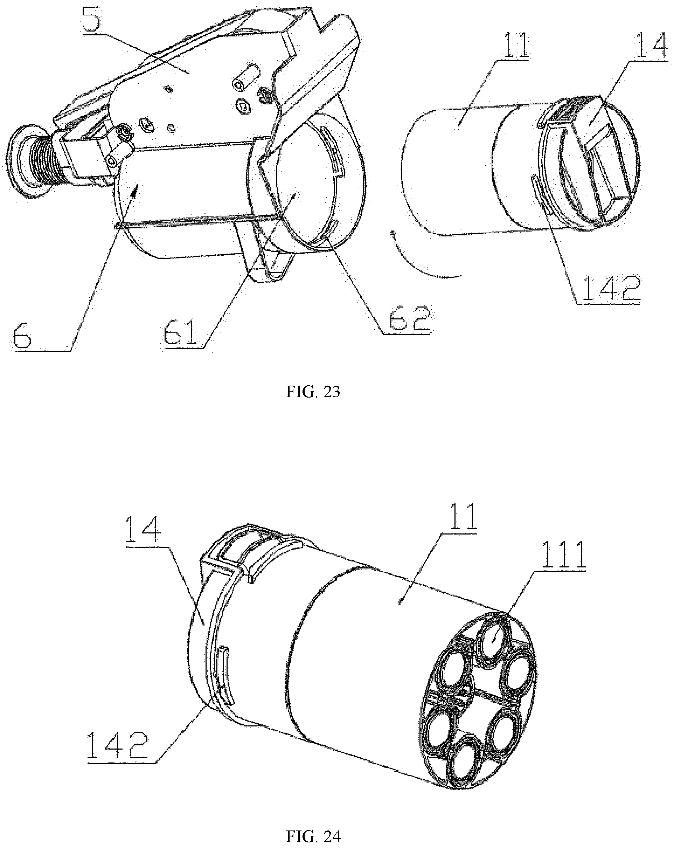

FIG. 23 is an exploded view of the test device provided in the fourth embodiment of the present invention;
FIG. 24 is a three-dimensional diagram of a test assembly of the test device provided in the fourth embodiment of the present invention;

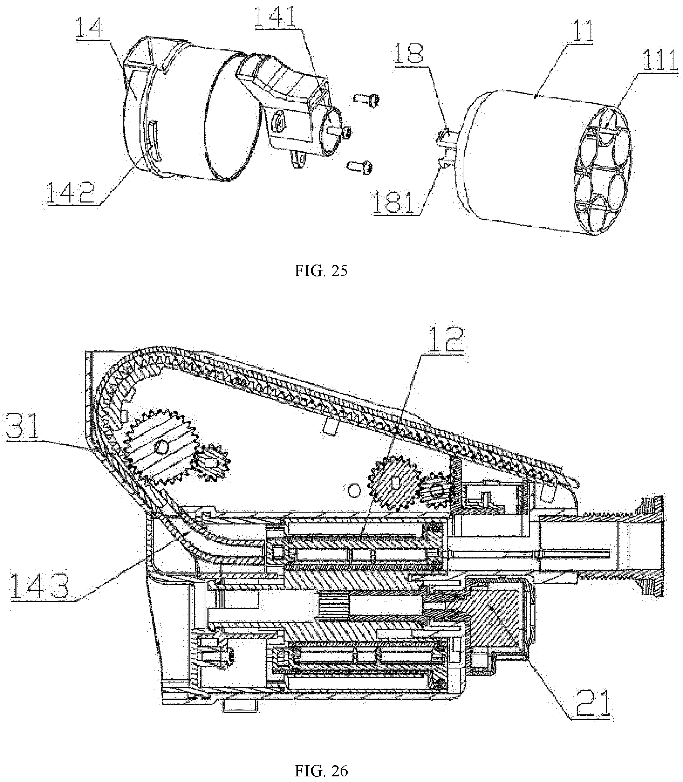

FIG. 25 is an exploded view of a test assembly of a test device provided in the fourth embodiment of the present invention;
FIG. 26 is a cross-sectional view of the test device provided in the fourth embodiment of the present invention;

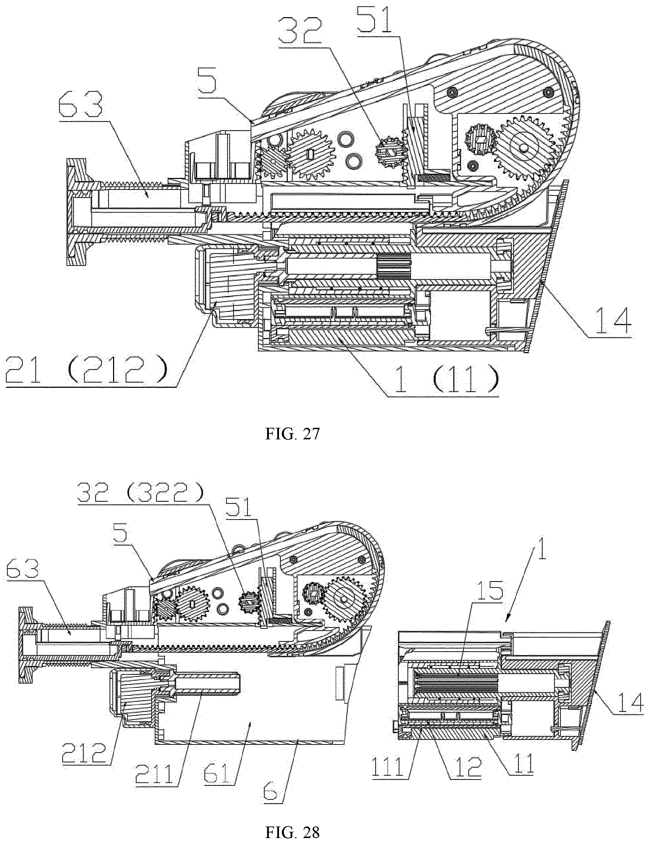

FIG. 27 is a first cross-sectional view of a test device provided in a fifth embodiment of the present invention (mounted with a test assembly);
FIG. 28 is a second cross-sectional view of the test device provided in the fifth embodiment of the present invention (with the test assembly removed);

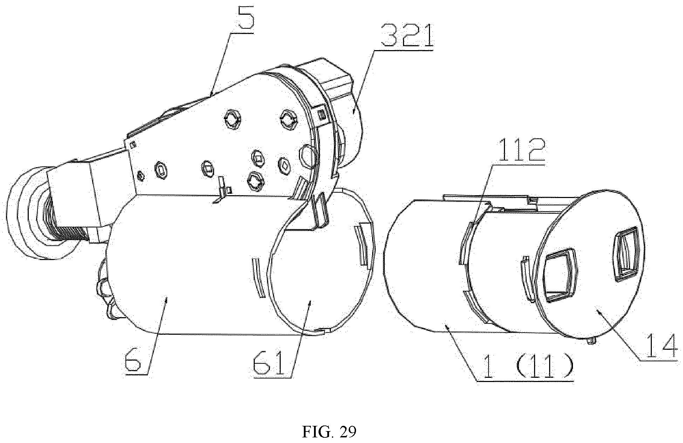

FIG. 29 is an exploded view of the test device provided in the fifth embodiment of the present invention;

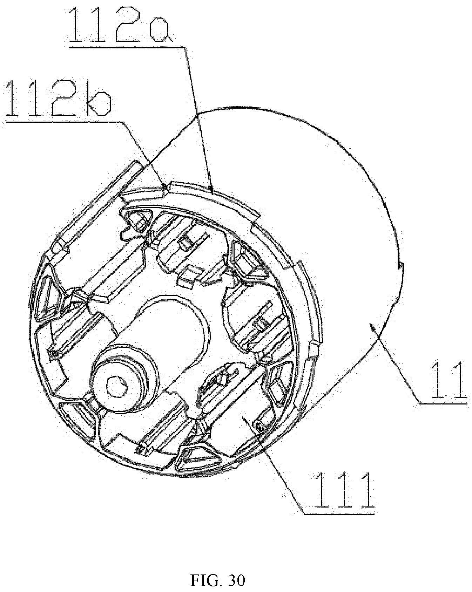

FIG. 30 is a three-dimensional diagram of a container body provided in the fifth embodiment of the present invention; and

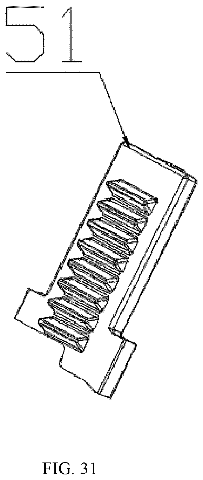

FIG. 31 is a three-dimensional diagram of a positioning member of the test device provided in the fifth embodiment of the present invention.

### Description of reference numerals:

Test assembly - 1;
Container body - 11; accommodating channel - 111; positioning portion - 112; front wall of the positioning groove - 112a; side wall of the positioning groove - 112b;
Test module - 12; inner cavity - 121; through channel - 122; support frame - 123; mounting groove - 1231; sealing member - 1232; hook - 1233; test paper - 124; groove - 125;
Plugging element - 13;
Rear cover - 14; second connecting portion - 141; fit portion - 142; yielding channel - 143;
Insertion hole - 15; limit rib - 151; clamping plate - 152;
Central cavity - 16; hanging groove - 161; anti-falling rib - 162; protrusion - 1621; yielding groove - 163;
Locking member - 17; guide cylinder - 171; boss - 1711; convex portion - 172; hook - 173; elastic member - 174;
First connecting portion - 18; inverted buckle - 181;
Driving mechanism - 21; first driving assembly - 211; limit groove - 2111; step - 2112; first power member - 212;
First transmission mechanism - 31; rack - 311; buckle - 3111; gear set - 312;
Second transmission mechanism - 32; driving motor - 321; second gear set - 322;
Toilet - 4; test hole - 41; accommodating chamber - 42;
Bracket - 5; positioning member - 51;
Base - 6; accommodating chamber - 61; limit portion - 62; test channel - 63.

### DETAILED DESCRIPTION

In order to make the objectives, technical solutions and advantages of the present invention clearer, the following clearly and completely describes the technical solutions in the embodiments of the present invention with reference to the accompanying drawings in the embodiments of the present invention. Apparently, the described embodiments are some but not all of the embodiments of the present invention. It should be understood that the specific embodiments described herein are merely used for interpreting the present invention, rather than limiting the present invention. All other embodiments obtained by those of ordinary skill in the art based on the embodiments in the present invention without any creative effort fall within the scope of protection of the present invention.

### First embodiment

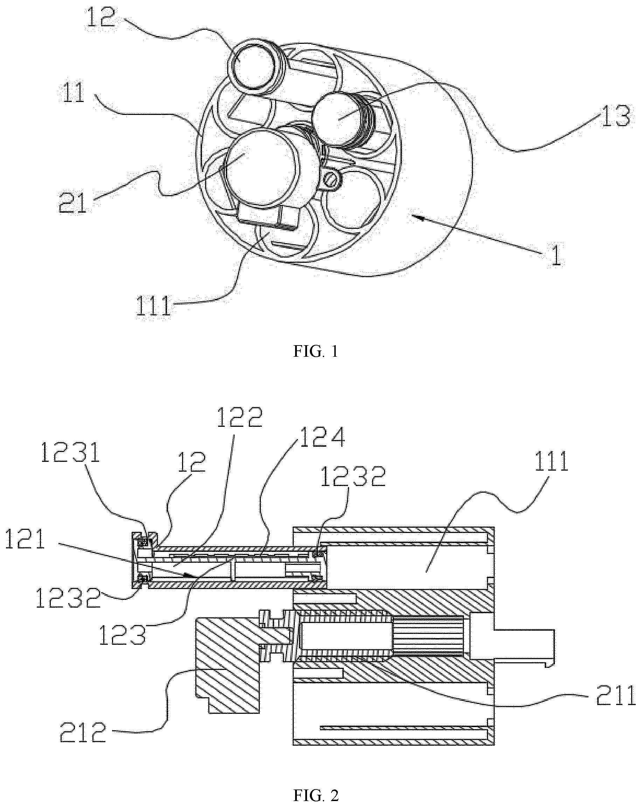

With reference to FIG. 1 to FIG. 4, the present invention first provides a test device. The test device includes a container body 11 and a test module 12 stored in the container body. The interior of the container body 11 is provided with a plurality of mutually independent accommodating channels 111. Several test modules 12 are provided and mounted in the accommodating channels 111 respectively (one/single test module 12 is mounted in each/single channel). Each test module 12 can independently extend out of the corresponding accommodating channel 111, that is, each test module 12 is in sliding fit with the corresponding accommodating channel 111 to extend out of the accommodating channel 111. The test module 12 includes a test element, the test element is used for test a liquid under test, and the test element includes a test tool, such as test paper 124. In other embodiments, the test tool may be other common test tools, such as a test card or a test pen. The test module may have an inner cavity 121, and the inner cavity 121 is used for storing the test element. Specifically, the container body 11 may be a shell, and the accommodating channel 111 includes two opposite ends and side walls connecting the two ends. In some embodiments, the side walls are connected into a whole (refer to annular side walls in FIGs. 1-4), that is, the cross-section of the accommodating channel 111 has a hollow closed shape, such as circular in FIGs. 1-4, or triangular, quadrilateral, pentagonal, hexagonal, octagonal, etc., as long as the accommodating channels can be independent of each other and be in sliding fit with the test modules 12. At least two test modules 12 may be mounted in the plurality of accommodating channels 111, that is, the test modules 12 are mounted in at least two accommodating channels 111, such that the user does not need to clean and discard the waste test module 12 after each test. The test module 12 is in sliding fit with the accommodating channel 111 that accommodates the test module to extend out of the accommodating channel 111.

In one embodiment, as shown in FIG. 2, the test module 12 includes a test kit with an inner cavity 121, two ends of a kit body of the test kit are throughly connected to form a through channel 122, the test element is provided in the test kit, and the test element includes test paper 124 and a support frame 123. The support frame 123 is connected to the through channel 122 in a sliding and sealing manner, such that the test element can extend out of the through channel 122 or retract into the through channel 122. The test paper 124 is laid on the support frame 123, such that the test paper 124 is in a closed environment before and after use, will not be affected with damp before use, and will not emit odors to affect the environment after use. Specifically, two ends of the support frame 123 are adapted to the height of the through channel 122. The two ends of the support frame 123 are further provided with mounting grooves 1231, a sealing member 1232 is mounted in the mounting groove 1231, and the sealing member 1232 may be a sealing ring, preferably a Y-shaped ring with good sealing property. The test paper 124 is laid on the support frame 123.

With reference to FIGs.1-4, the aforementioned test device may further include a driving mechanism 21, where the driving mechanism 21 is in transmission fit with the container body 11 to drive the container body 11 to move. The driving mechanism 21 may include a first driving assembly 211 and a first power member 212, where the first power member 212 provides driving force for the first driving assembly 211. The aforementioned test device may further include a plugging element 13. Specifically, when test is performed, the test module 12 extends out of the accommodating channel 111 for test through the test element. When no test is performed, the plugging element 13 extends out of the accommodating channel 111 for plugging.

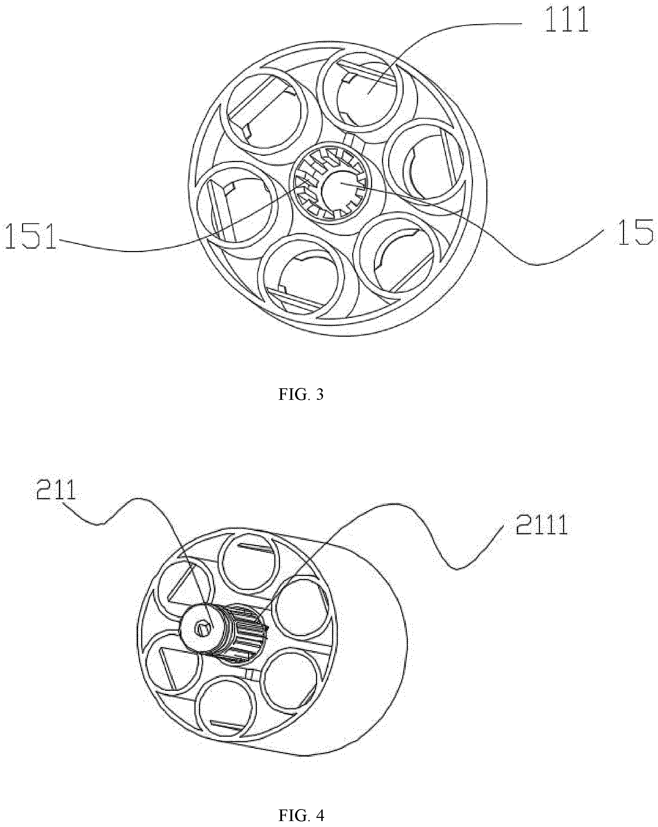

With reference to FIGs. 1-4, in a preferred embodiment, the container body 11 is cylindrical, the accommodating channel 111 penetrates along an axis of the container body 11, and the plurality of accommodating channels 111 are arranged sequentially along a circumference of the container body 11. The first driving assembly 211 includes a driving shaft, and the first power member 212 is a motor. The container body 11 is provided with an insertion hole 15, and the driving shaft is in circumferential limiting fit with the insertion hole 15. More specifically, as shown in FIG. 3 and FIG. 4, limit ribs 151 are provided in the insertion hole 15, the driving shaft is provided with limit grooves 2111, and the limit ribs 151 are adapted to the limit grooves 2111 to ensure a stable connection between the driving shaft and the container body 11, with good transmission effect and fastening effect. It is easy to conceive that the positions of the limit ribs 151 and the limit grooves 2111 can also be interchanged (not shown), that is, the limit grooves are provided in the insertion hole 15, and the limit ribs are provided on the driving shaft. The insertion hole 15 is located in a central position of the container body 11, such that the driving mechanism 21 is mounted more stably, and it is more stable for the driving mechanism to move and drive the container body 11 to move, and the container body can be better adapted to a test hole 41 of a toilet. In this embodiment, the driving mechanism 21 drives the container body 11 to rotate, such that the accommodating channels 111 also rotate with the container body 11 (thereby achieving the switching of a target accommodating channel inside the container body 11, where the target accommodating channel is a channel corresponding to the position of the liquid under test). Because the container body 11 is cylindrical, its rotation effect is optimal. In other embodiments, the container body 11 may alternatively be in the shape of a prism, and the more sides of the prism, the closer the prism is to the cylindrical shape, and the better the effect.

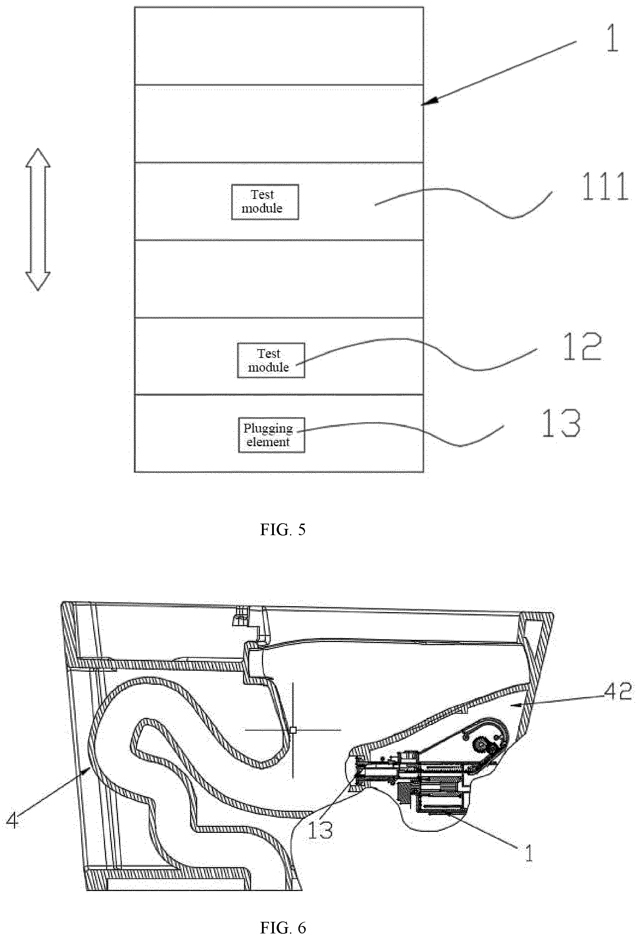

In another embodiment, the driving mechanism 21 drives the container body 11 to slide to a preset position. Specifically, as shown in FIG. 5, the container body 11 has a plurality of independent accommodating channels 111 inside, and the plurality of accommodating channels 111 are arranged sequentially according to the sliding direction. The driving mechanism 21 drives the container body 11 to slide up and down, so the plurality of accommodating channels 111 are arranged sequentially in the vertical direction. In other embodiments, the plurality of accommodating channels 111 may alternatively be arranged sequentially in a horizontal direction or in other directions, as long as their arrangement direction is consistent with the direction in which the driving mechanism 21 drives the container body 11 to slide.

In one embodiment, a first transmission mechanism 31 is further included, and the first transmission mechanism 31 has a transmission member capable of driving the test module 12 to move. Specifically, the transmission member can extend into or exit from the accommodating channel 111 for transmission connection with the test module 12 or the plugging element 13, so as to drive the test module 12 or plugging element 13 to extend out of the accommodating channel 111.

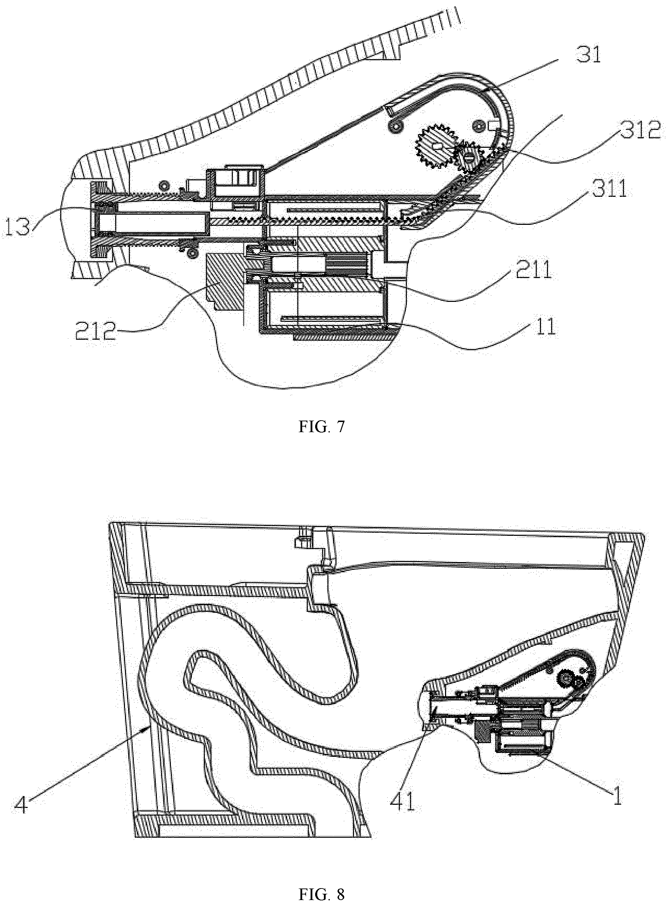

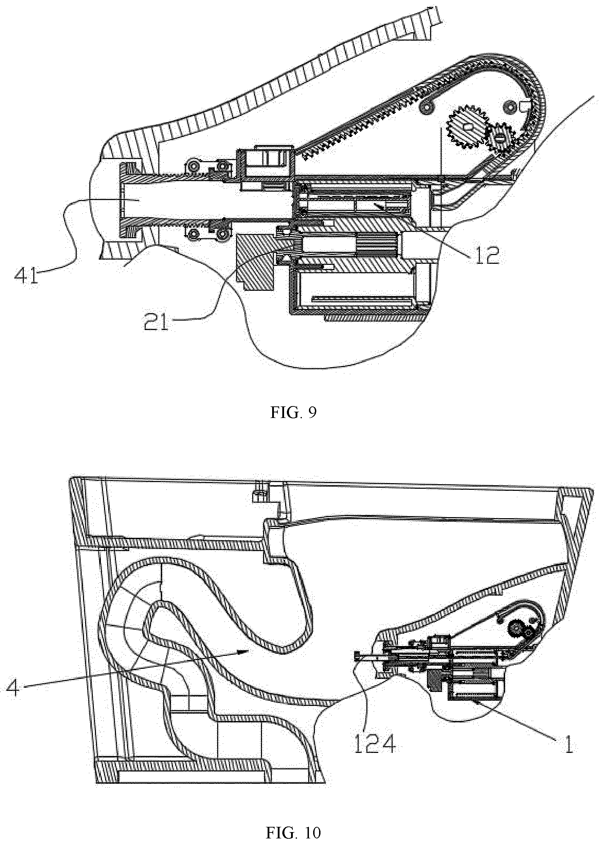

In one embodiment, with reference to FIGs. 6-11, the transmission member is a rack 311, the rack 311 is in separable fit with the test module 12 or the plugging element 13, and the first transmission mechanism 31 further includes a gear set 312 that meshes with the rack 311 and a motor (not shown) that drives the gear set 312.

The aforementioned test device for the liquid under test further includes a base, and the container body 11 is detachably mounted on the base.

In one specific embodiment, the test device includes a driving mechanism 21 and a first transmission mechanism 31, where the driving mechanism is in transmission connection with the container body 11 to drive the container body 11 to move. Under the joint action of the driving mechanism 21 and the first transmission mechanism 31, the movement of the container body 11 can be achieved, and the test module 12 or the plugging element 13 extends out of the accommodating channel 111.

In another embodiment, the test assembly 1 also includes a driving mechanism 21 and a first transmission mechanism 31, where the first transmission mechanism 31 can drive the test module 12 or the plugging element 13 to move. But unlike the previous embodiment, the driving mechanism 21 is not connected to the container body 11 (not shown), but is in transmission connection with the first transmission mechanism 31 through a first driving assembly. The movement (such as rotation or sliding) of the driving mechanism 21 drives the movement of the first transmission mechanism 31, thereby switching the target in transmission connection with the first transmission mechanism 31, that is, switching between different test modules 12 or between the test module 12 and the plugging element 13.

The present invention further provides a toilet 4. The toilet 4 includes the aforementioned test assembly 1 for a liquid under test. With reference to FIGs. 6-11, a toilet bowl of the toilet 4 is provided with a test hole 41 that allows a test tool to extend out to test the liquid under test. Specifically, a front wall of the toilet may be provided with an accommodating chamber 42, the test assembly 1 is mounted in the accommodating chamber 42, and the accommodating chamber 42 is in communication with the toilet bowl through the test hole 41. The position of the test hole 41 corresponds to the test module 12 or the plugging element 13. When test is performed, the driving mechanism 21 drives the container body 11 to move, such that the test module 12 corresponds to the test hole 41. When no test is performed, the driving mechanism 21 drives the container body 11 to move, such that the plugging element 13 corresponds to the test hole 41.

Further, when the test module 12 corresponds to the test hole 41, the first transmission mechanism 31 drives the test element to extend out from the test hole 41 for test; when the plugging element 13 corresponds to the test hole 41, the first transmission mechanism 31 drives the plugging element 13 to extend out to plug the test hole 41.

As shown in FIG. 6 to FIG. 11, as an example, a process of using the aforementioned toilet to test urine through urination may include the following steps:
When the test module 12 is not used, the plugging element 13 extends out of the accommodating channel 111 to seal the test hole 41;
When the user needs to test the liquid under test, the first transmission mechanism 31 is operated to drive the plugging element 13 back into the accommodating channel 111, the driving mechanism 21 is operated to drive the container body 11 to move, the initial state that the plugging element 13 corresponds to the test hole 41 is switched to the state that the test module 12 corresponds to the test hole 41 (communication), then the first transmission mechanism 31 is operated to drive the test element (the support frame 123 and the test paper 124) to extend a distance, the first transmission mechanism 31 further drives the test element and the test kit together to continue to extend out of the accommodating channel 111, and the test element extends from the test hole 41 into the toilet bowl to receive urine for test;
After the test is completed, the first transmission mechanism 31 is operated to drive the used test element back into the test kit, and to further drive the test module 12 back into the original accommodating channel 111 (or the used test module does not retract, but is directly discarded); the driving mechanism 21 is operated to drive the container body 11 to move, the state that the test module 12 corresponds to the test hole 41 (communication) is switched to the state that the plugging element 13 corresponds to the test hole 41, and then the first transmission mechanism 31 is operated to drive the plugging element 13 to extend out of the accommodating channel 111 to plug the test hole 41.

The present invention further provides a method for testing a liquid under test, which includes the following process:
Provide the container body 11 for storing test modules 12, where the container body 11 is provided with a plurality of mutually independent accommodating channels 111 inside, the test module is mounted in the accommodating channel 111, and the test module 12 is provided with a test element for test a liquid under test; the first transmission mechanism 31 drives the test module 12 to extend out of the corresponding accommodating channel 111; the first transmission mechanism 31 moves or the container body 11 moves, and where the first transmission mechanism 31 can drive the test module 12 to extend out of the corresponding accommodating channel 111.

In one embodiment, when test is performed, the container body 11 moves to a position where one test module 12 corresponds to the test hole 41 of the toilet, and the first transmission mechanism 31 drives the test module 12 to extend out of the corresponding accommodating channel 111;
After the test is completed, the test module 12 retracts into the original accommodating channel 111; or the test module 12 is discarded.

The test method in the above embodiment may further include the following process:
After the test is completed, the container body 11 moves to a position where the next test module 12 corresponds to the test hole 41 of the toilet;
When the test is performed again, the next test module 12 extends out of the corresponding accommodating channel 111;
After the test is completed again, the test module 12 retracts into the original accommodating channel 111; or the test module 12 is discarded.

Alternatively, the test method in the above embodiment may further include the following process:
When the test is performed again, the container body 11 moves to a position where the next test module 12 corresponds to the test hole 41, and another test module 12 extends out of the corresponding accommodating channel 111;
After the test is completed again, the test module 12 retracts into the original accommodating channel 111; or the test module 12 is discarded.

### Second embodiment

The basic principle of this embodiment is the same as that of the first embodiment, except that the container body in this embodiment is provided with an anti-falling structure, such that the test module and the container body can be securely stored on the container body.

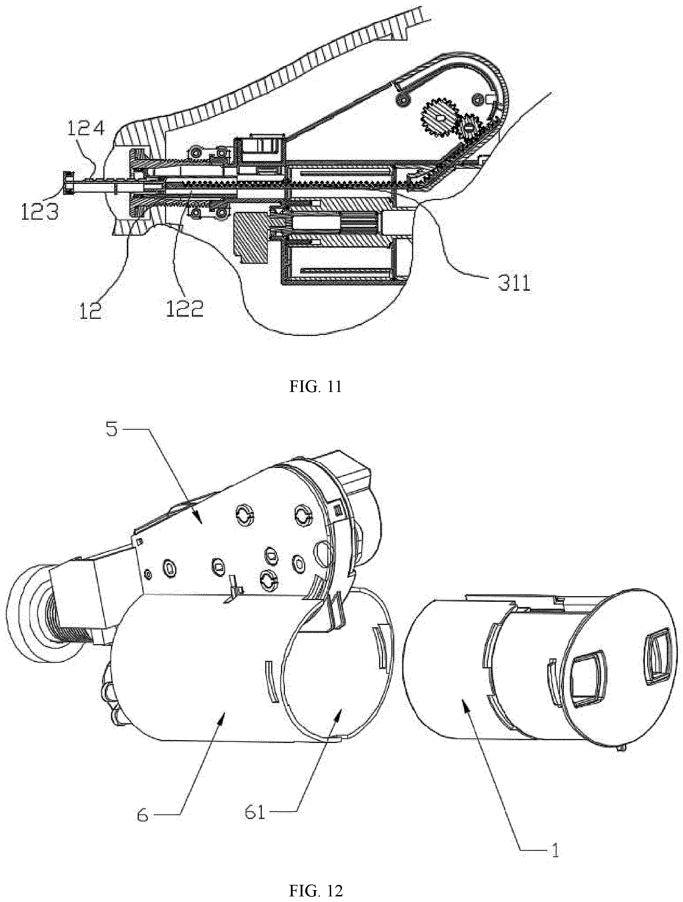

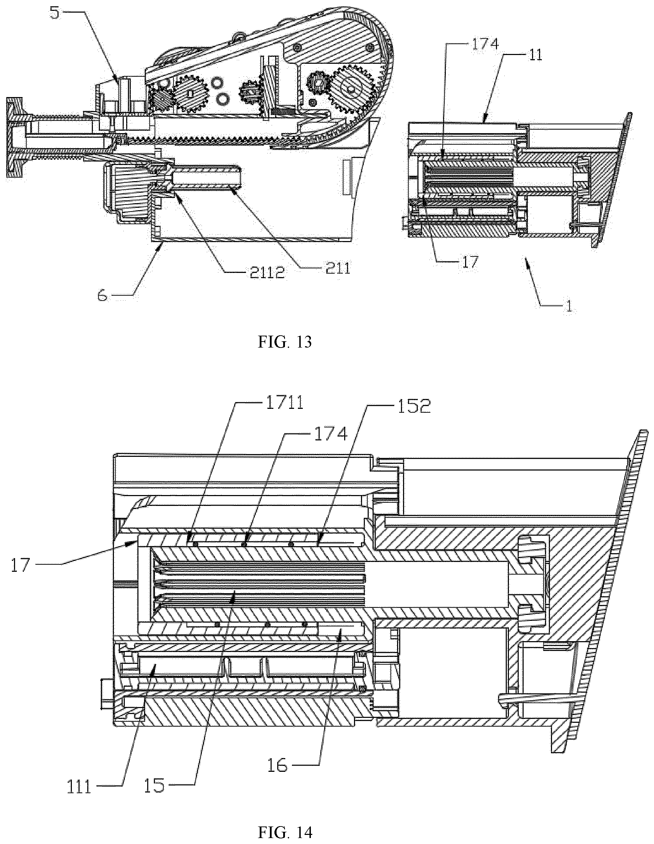

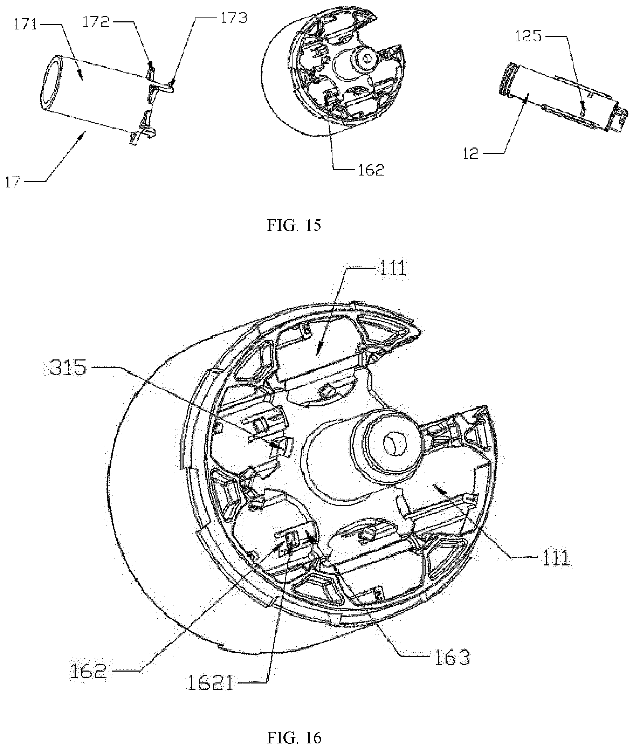

With reference to FIG. 12 to FIG. 18, the test module 12 is movable between a test position and a storage position. The accommodating channel 111 is provided with a limit member, and the limit member is movable between a limiting position and a yielding position. When the limit member moves to the limiting position, the limit member is in limiting fit with the test module 12 to limit the test module 12 in the storage position. When the limit member moves to the yielding position, the limit member is in yielding fit with the test module 12, and the test module 12 can move between the test position and the storage position. The limit member is in limiting/yielding fit with the test module 12, such that the test module 12 can be securely stored on the container body 11, which prevents the test module 12 from falling off without interfering with the normal ejection and use of the test module 12.

The anti-falling structure further includes a locking member 17, and the locking member 17 is movably arranged on the container body 11. When the locking member 17 moves to a locking position, the locking member 17 limits the limit member to the limiting position, thereby fixing the test module 12 to the storage position. When the locking member 17 moves to an unlocking position, the locking member 17 releases the limiting of the limit member, such that the limit member can move to the yielding position, and the limit member is in yielding fit with the test module 12 without interfering with the normal test of the test module 12.

The test device further includes a bracket 5 and a base 6 arranged on the bracket 5, where an accommodating chamber 61 is provided in the base 6. The container body 11 is detachably mounted on the base 6. When the container body 11 is assembled with the base 6 in place, the locking member 17 moves from the locking position to the unlocking position under the action of the base 6. That is, when the container body 17 is not assembled to the base 6 or is not assembled in place, the locking member 17 is always in the locking position, thereby limiting the limiting member to the limiting position, such that the limit member limits the test module 12 to the storage position, to avoid the risk of falling of the test module 12. After the container body 11 is assembled onto the base 6, under the action of the base 6, the locking member 17 releases the limiting of the limiting member, such that the limit member is in yielding fit with the test module 12 to avoid interfering with the normal operation of the test module 12. It is not difficult to see that the anti-falling structure in this embodiment does not affect the normal test of the test module 12, and can effectively avoid the risk of falling of the test module 12 from the container body 11 before the container body 11 is assembled to the base 6.

A first power member 212 and a first driving assembly 211 are provided on the base 6. In this embodiment, the first driving assembly 211 is a driving shaft, the container body is provided with a central cavity 16, the accommodating channel 111 is used for placing the test module 12, the central cavity 16 further includes a sleeve with an insertion hole 15, and the insertion hole 15 is used for docking with the driving shaft when the test assembly 1 is assembled with the base 6. A step 2112 is provided on the peripheral side of the end of the driving shaft away from the container body 11, and the step 2112 is adapted to the end of the locking member 17 away from the convex portion 172.

The limit member is an anti-falling rib 162 arranged on a cavity wall of the accommodating channel 111, and the test module 12 is provided with a groove 125 in limiting fit with the anti-falling rib 162. It can be understood that the anti-falling rib 162 has a deformation quantity between the limiting position and the yielding position. Specifically, the anti-falling rib 162 is further provided with a protrusion 1621 extending towards the axis of the accommodating channel 111. When the locking member 17 moves to the locking position, the anti-falling rib 162 is in limiting fit with the test module 12, and the protrusion 1621 gets stuck into the groove 125, thereby limiting and storing the test module 12 in the accommodating channel 111. When the locking member 17 moves to the unlocking position, the anti-falling rib 162 is in yielding fit with the test module 12, and the test module 12 is moved to squeeze the protrusion 1621, such that the anti-falling rib 162 deforms towards the central cavity 16 to yield the test module 12, and the test module 12 can move to the test position. It should be noted that, during the yielding process, the protrusion 1621 abuts against an outer wall of the test module 12, and small frictional force produced between the protrusion 1621 and the test module 12 will not hinder the sliding of the test module 12.

The locking member 17 includes a guide cylinder 171 and a convex portion 172 arranged on the guide cylinder 171, the guide cylinder 171 is sleeved on an outer wall of the sleeve, the convex portion 172 is arranged at an end of the guide cylinder 171 and extends away from the axis of the guide cylinder 171, and the shape of the convex portion 172 is adapted to the shape of the inner wall of the central cavity 16.

When the guide cylinder 171 moves to the locking position, the convex portion 172 abuts against the anti-falling rib 162 to limit the deformation of the anti-falling rib 316, and the protrusion 1621 is clamped in the groove 125. When the guide cylinder 171 moves to the unlocking position, the convex portion 172 separates from the anti-falling rib 162 to release the deformation limiting of the anti-falling rib 162, such that the protrusion 1621 can separate from the groove 125. In this embodiment, a yielding groove 163 is formed axially on the side, provided with the protrusion 1621, of the anti-falling rib 162 on the cavity wall of the accommodating channel 111. When the convex portion 172 moves to the yielding groove 163, the locking member 17 releases the deformation limiting of the anti-falling rib 162.

The outer wall of the sleeve is sleeved with an elastic member 174, and the elastic member 174 is located in a gap between the guide cylinder 171 and the sleeve. The locking member 17 is provided with a first abutting portion, the outer wall of the sleeve is provided with a second abutting portion, and two ends of the elastic member 174 abut against the first abutting portion and the second abutting portion. In this embodiment, the first abutting portion is a boss 1711 that is arranged at the end of the guide cylinder 171 away from the convex portion 172 and faces the insertion hole 15, the second abutting portion is a clamping plate 152 that is arranged on the outer wall of the sleeve and extends radially along the insertion hole 15, and the two ends of the elastic member 174 abut against the boss 1711 and the clamping plate 152. The guide cylinder 171 is further provided with a hook 173, a hanging groove 161 is provided on the cavity wall of the central cavity 16, the hanging groove 161 is formed axially, and the hook 173 can move axially in the hanging groove 161. That is, after the locking member 17 is assembled with the container body 11, the locking member 17 can also move axially within a certain range, depending on the axial length of the hanging groove 161. When the hook 173 is inserted into the hanging groove 161, the distance between the boss 1711 and the clamping plate 152 is less than the original length of the elastic member 174. In this embodiment, the hook 173 is arranged at the same end as the convex portion 172.

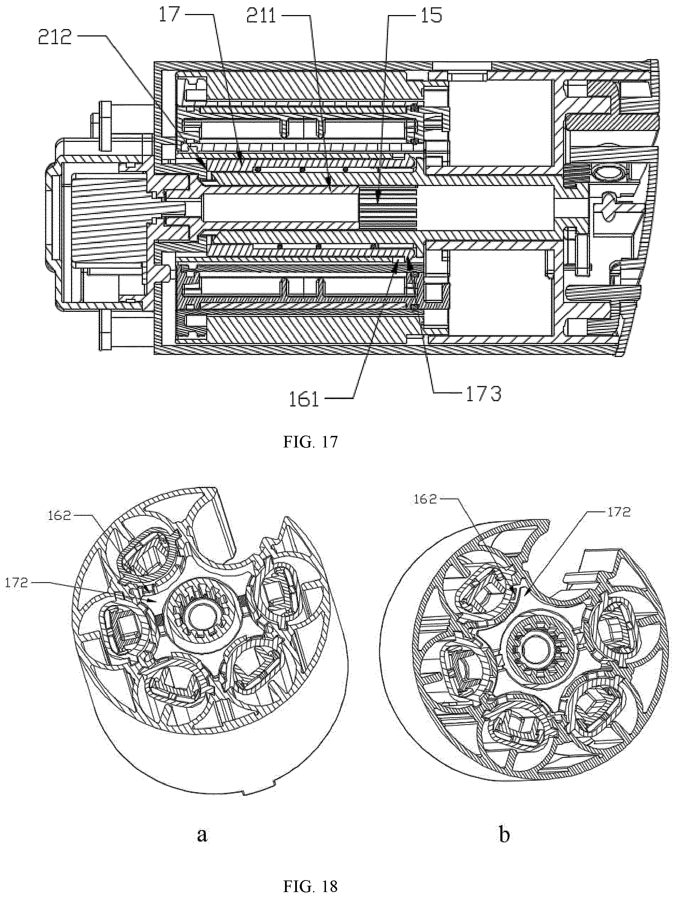

The mounting and working principle of the anti-falling structure are as follows:
① The test module 12 is placed in the accommodating channel 111, and the test module 12 squeezes the protrusion 1621, such that the anti-falling rib 162 deforms towards the central cavity 16 to yield the test module 12 until the test module 12 is mounted in place with the container body 11. At this time, the protrusion 1621 gets stuck into the groove 125, and the anti-falling rib 162 recovers its deformation. However, in this state, the limiting of the test module 12 by the anti-falling rib 162 is not firm, and the test module 12 may move by slight drag/push.
② The end, provided with the convex portion 172, of the locking member 17 extends into the central cavity 16, the convex portion 172 moves in contact with the inner wall of the central cavity 16 until the hook 173 of the locking member 17 gets stuck into the hanging groove 161, the elastic element 174 is compressed, and the elastic force of the elastic element 174 enables the guide cylinder 171 to have a tendency to move away from the convex portion 172. The hook 173 is then clamped and limited in the hanging groove 161, such that the guide cylinder 171 is fixed in this position, that is, the limiting position. At this time, the convex portion 172 just abuts against the anti-falling rib 162, thereby preventing deformation of the anti-falling rib 162, as shown in FIG. 18a. Then, the test module 12 is firmly stored in the accommodating channel 111.
③ The test assembly 1 is assembled into the base 6, and the step 2112 abuts against the end of the guide cylinder 171 to push the guide cylinder 171 axially until the test assembly 1 is assembled in place with the base 6. At this time, the step 2112 presses against the locking member 17 to overcome the elastic force of the elastic member 174, and the convex portion 172 moves from the locking position of abutting against the anti-falling rib 162 to the yielding groove 163, that is, the unlocking position, thereby releasing the deformation limiting of the anti-falling rib 162, as shown in FIG. 18b.

### Third embodiment

The principle of this embodiment is the same as that of the first embodiment or the second embodiment, and improvements are made on the basis of the first embodiment or the second embodiment. In this embodiment, one end of the test module 12 is provided with a connecting portion, and the transmission member is provided with a clamping portion. When the container body 11 drives the test module 12 to move to a first position, the clamping portion separates from the connecting portion, such that the transmission member separates from the test module 12. When the container body 11 drives the test module 12 to move to a second position, the connecting portion is in limiting fit with the clamping portion, such that the transmission member is in transmission fit with the test module 12.

The container body 11 is rotatably arranged on the base 6, and the container body 11 rotates within a preset angle to drive the test module 12 to move between the first position and the second position. The container body 11 is arranged on the base 6 in a sliding manner, and the container body 11 slides within a preset distance to drive the test module 12 to move between the first position and the second position.

In this embodiment, the transmission member is a rack 311, and a motor (not shown) and a gear set 312 that meshes with the rack 311 provide power for the rack 311.

In this embodiment, when the rack 311 is in transmission fit with the test module 12, and during the process that the connecting portion is in limiting fit with the clamping portion within the accommodating channel 111, the connecting portion and the clamping portion cannot be separated under the limiting effect of the cavity wall of the accommodating channel 111.

In other embodiments, the connecting portion and the clamping portion cannot be separated under the limiting action of the limit ribs in the accommodating chamber.

In this embodiment, one of the connecting portion and the clamping portion is a buckle 3111, and the other of the connecting portion and the clamping portion is a hook 1233.

In this embodiment, the buckle 3111 is a concave portion with a side opening, and the hook 1233 is inserted into the buckle 3111 from the side opening.

A specific working process of this embodiment is as follows:
The test module 12 is inserted into the accommodating channel 111 of the container body 11, the first power member 212 drives the container body 11 to rotate forward, the hook 1233 of the test module 12 is fastened into the buckle 3111 along one side of the rack 311, and then the rack 311 pushes the test module 12 to extend out of the accommodating channel 111. After urination, the rack 311 pulls back the test module 12. Due to the abutting limiting fit between the hook 1233 and the buckle 3111 in this solution, the hook 1233 and the buckle 3111 are less prone to elastic deformation. Therefore, the hook 1233 and the buckle 3111 are not prone to detachment, and the hook 1233 and the buckle 3111 cannot be separated in the accommodating channel 111, making them even more difficult to detach. When the hook 1233 is exposed outside the accommodating channel 111, the container body 11 is driven by the motor to rotate reversely, such that the hook 1233 and the buckle 3111 are separated, thereby achieving detachment.

### Fourth embodiment

The principle of this embodiment is the same as that of the first embodiment, the second embodiment, or the third embodiment, and improvements are made on the basis of the first embodiment, the second embodiment, or the third embodiment. In this embodiment, the test device includes:
a bracket 5 and a test assembly 1, where the bracket is provided with a base 6, and an accommodating cavity 61 is formed on the base 6. The test assembly 1 is mounted in the base 6, the test assembly 1 includes a container body 11 and a rear cover 14, a plurality of test modules 12 are mounted in the container body 11, and the rear cover 14 is movable between a locking position and an unlocking position. When the rear cover 14 moves to the locking position, the rear cover 14 limits the container body 11 in the base 6. When the rear cover 14 moves to the unlocking position, the container body 11 can exit from the base 6.

In this embodiment, the rear cover 14 is movably mounted on the container body 11. When the rear cover 14 moves to the locking position, the rear cover 14 is in limiting fit with the base 6 to limit the container body 11 in the base 6. When the rear cover 14 moves to the unlocking position, the rear cover 14 is in yielding fit with the base 6, such that the container body 11 can exit from the base 6.

In other embodiments, the rear cover 14 is movably mounted on the base 6. When the rear cover 14 moves to the locking position, the rear cover 14 is in limiting fit with the container body 11 to limit the container body 11 in the base 6. When the rear cover 14 moves to the unlocking position, the rear cover 14 is in yielding fit with the container body 11, such that the container body 11 can exit from the base 6.

In this embodiment, the container body 11 is provided with a first connecting portion 18, the rear cover 14 is provided with a second connecting portion 141, and the first connecting portion 18 is in axial limiting fit and circumferential yielding fit with the second connecting portion 141 along the container body 11.

In this embodiment, one of the first connecting portion 18 and the second connecting portion 141 is a pivot hole, and the other of the first connecting portion 18 and the second connecting portion 141 is a pivot shaft. Specifically, the first connecting portion 18 is a pivot shaft, and the second connecting portion 141 is a pivot hole.

In this embodiment, the pivot shaft is provided with an inverted buckle 23 in clamping fit with the pivot hole.

In this embodiment, the base 6 includes an accommodating chamber 61, the test assembly 1 is mounted in the accommodating chamber 61, a front end of the accommodating chamber 61 is provided with a front opening that allows the test module 12 to extend out, a rear end of the accommodating chamber 61 is provided with a rear opening and a limit portion 62, the rear cover 14 is arranged on the side of the accommodating chamber 61 near the rear opening, and the rear cover 14 is provided with a fit portion 142. When the rear cover 14 moves to the locking position, the fit portion 142 is in limiting fit with the limit portion 62, and then the container body 11 is limited in the accommodating chamber 61 by the rear cover 14. When the rear cover 14 moves to the unlocking position, the fit portion 142 is in yielding fit with the limit portion 62, and then both the rear cover 14 and the container body 11 can exit the accommodating chamber 61 from the rear opening. The fit between the container body 11 and the base 6 is not limited to clamping limit, and may also be axial limiting fit along the container body 11, etc., which will not be repeated here.

In this embodiment, a first transmission mechanism 31 is further included, the first transmission mechanism 31 includes a transmission member in transmission fit with the test module 12, and the rear cover 14 further has a yielding channel 143; when the rear cover 14 moves to the locking position, the transmission member can extend into the yielding channel 143 to be in transmission fit with the test module 12.

In this embodiment, the container body 11 is provided with a plurality of accommodating channels 111, and the plurality of test modules 12 are mounted in the accommodating channels 111 respectively.

In this embodiment, the container body 11 is movably mounted in the base 6, a driving mechanism 21 is further included, and the driving mechanism 21 includes a first driving assembly 211 in transmission fit with the container body 11.

In this embodiment, the container body 11 is rotatably arranged and provided with an insertion hole 15. The first driving assembly 211 is a driving shaft that extends into the insertion hole 15. The driving shaft is in circumferential limiting fit with the insertion hole 15 to drive the container body 11 to rotate.

In this embodiment, the driving mechanism 21 further includes a first power member 212 that drives the driving shaft to rotate, and the first power member 212 is a motor.

In this embodiment, the transmission member is a rack 311, and the first transmission mechanism 31 further includes a motor that drives the rack 311 to move and a gear set 312.

A specific working process of this embodiment is as follows:
When the rear cover 14 moves to the locking position, the fit portion 142 is in limiting fit with the limit portion 62, and then the container body 11 is limited in the accommodating chamber 61 by the rear cover 14. When the rear cover 14 moves to the unlocking position, the fit portion 142 is in yielding fit with the limit portion 62, and then both the rear cover 14 and the container body 11 can exit the accommodating chamber 61 from the opening.

### Fifth embodiment

The principle of this embodiment is the same as that of the first embodiment, the second embodiment, the third embodiment, or the fourth embodiment, and improvements are made on the basis of the first embodiment, the second embodiment, the third embodiment, or the fourth embodiment. In this embodiment, the test device includes:
a bracket 5, where the bracket 5 is provided with a base 6, and the base 6 has an accommodating chamber 61;
a test assembly 1 detachably mounted in the accommodating chamber 61;
a driving mechanism 21 used for driving the test assembly 1 to move;
a positioning member 51 movably arranged on the bracket 5, where when the positioning member 51 moves to a first position, the positioning member 51 can be in limiting fit with the test assembly 1; when the positioning member 51 moves to a second position, the positioning member 51 can be in yielding fit with test assembly 1;
a second transmission mechanism 32 used for driving the positioning member 51 to move; and
a main control board electrically connected to the driving mechanism 21 and the second transmission mechanism 32 respectively to control the operation of the driving mechanism 21 and the second transmission mechanism 32.

In this embodiment, the test assembly 1 is mounted in or exits from the accommodating chamber 61 in a direction parallel to its central axis, and the positioning member 51 moves in a direction perpendicular to the central axis of the test assembly 1.

In this embodiment, the base 10 is provided with a test channel 63, the test assembly 1 includes a container body 11 movably arranged in the accommodating chamber 61, the container body 11 includes a plurality of accommodating channels 111 mounted with test modules 12, the container body 11 switches to move along the accommodating chamber 61 to ensure that each accommodating channel 111 corresponds to the test channel 63 respectively, and the driving mechanism 21 is in transmission fit with the container body 11 to drive the container body 11 to move in the switching direction.

In this embodiment, the periphery of the container body 11 is provided with a positioning portion 112, and the positioning member 51 is in limiting fit with the positioning portion 112 in axial and circumferential directions of the test assembly 1.

In this embodiment, the positioning portion 112 includes a plurality of positioning grooves arranged at intervals in a circumferential direction of the container body 11, and the positioning member 51 forms an axial limiting fit with front walls 112a of the positioning grooves to limit the test assembly 1 from exiting the accommodating chamber 61; the positioning member 51 forms a circumferential limiting fit with side walls 112b of the positioning grooves to position the movement of the container body 11.

In this embodiment, the driving mechanism 21 includes a rotating motor (first power member) 212 and a driving shaft (first driving assembly) 211 arranged at an output end of the motor, the test assembly 1 is rotatably arranged in the accommodating chamber 61, the test assembly 1 is provided with an insertion hole 15 in insertion fit with the driving shaft 211, and the driving shaft 211 is in circumferential limiting fit with the insertion hole 15.

In this embodiment, the second transmission mechanism 32 includes a driving motor 321 and a second gear set 322 arranged at an output end of the motor, the positioning member 51 is a baffle, and one side of the baffle is provided with teeth matching the gear set 312.

In this embodiment, a rear end of the accommodating chamber 61 is provided with an opening, a rear cover 14 rotatably connected to the container body 11 is further included, and the rear cover 14 is detachably and fixedly mounted at the opening.

A specific working process of this embodiment is as follows:
When test is performed, the rotating motor 31 drives the driving shaft 211 to rotate, and the driving shaft 211 fits with the insertion hole 15 to drive the container body 11 to rotate. Meanwhile, the driving motor 321 drives the second gear set 322 to rotate, the second gear set 322 fits with the teeth of the positioning member 51 to drive the positioning member 51 to move downwards, the positioning member 51 fits with the positioning portion 112 (the side walls 112b of the positioning grooves) to limit the container body 11 to the position where the accommodation channel 111 corresponds to the test channel 63, and the positioning member 51 fits with the positioning portion 112 (the front walls 112a of the positioning grooves) to limit the container body 11 in the accommodating chamber 61, thereby preventing the test assembly 1 from being accidentally disassembled during the test process.

In other embodiments, the test assembly 1 is provided with a third abutting portion. When the positioning member 51 moves to a third position, the positioning member 51 is in limiting fit with the third abutting portion to limit the test assembly 1 along an axis of the test assembly 1. Specifically, the third abutting portion may be a convex ring arranged along a circumference of the test assembly 1 (container body 11).

In other embodiments, the test assembly 1 is provided with a fourth abutting portion. When the positioning member 51 moves to the third position, the positioning member 51 is in limiting fit with the fourth abutting portion to limit the test assembly 1 along a circumference of the accommodating chamber 61. The fourth abutting portion is a plurality of bosses arranged at intervals along the circumference of the test assembly 1 (container body 11).

The above explanation illustrates and describes the preferred embodiments of the present invention. It should be understood that the present invention is not limited to the form disclosed herein, should not be regarded as an exclusion of other embodiments, can apply to various other combinations, modifications and environments, and can be modified within the scope of conception of the present invention through the foregoing teachings or technologies or knowledge in related fields. Any modifications and changes made by those skilled in the art without departing from the spirit and scope of the present invention should fall within the protection scope of the appended claims of the present invention.

## Claims

1. A test device, comprising a test module, and further comprising a container body used for storing the test module, wherein
the interior of the container body is provided with a plurality of mutually independent accommodating channels; and
the test module comprises a test element, a liquid under test is tested by means of the test element, several the test modules are provided and mounted in the accommodating channels respectively, and each test module can independently extend out of the corresponding accommodating channel.

2. The test device according to claim 1, further comprising a first transmission mechanism, wherein the first transmission mechanism has a transmission member capable of driving the test module to move.

3. The test device according to claim 2, further comprising a driving mechanism, wherein the container body is movable, and the driving mechanism has a first driving assembly in transmission fit with the container body.

4. The test device according to claim 3, wherein the container body is rotatable, the container body is cylindrical, the accommodating channels penetrate along an axis of the container body, and the accommodating channels are arranged sequentially along a circumference of the container body.

5. The test device according to claim 4, wherein the first driving assembly comprises a driving shaft, the container body is provided with an insertion hole, and the driving shaft is in circumferential limiting fit with the insertion hole, so as to drive the container body to rotate by the driving shaft; one of the driving shaft and the insertion hole is provided with limit ribs, the other of the driving shaft and the insertion hole is provided with limit grooves, and the limit ribs are adapted to the limit grooves; the insertion hole is located at a center position of the container body.

6. The test device according to claim 3, wherein the driving mechanism further comprises a first power member, the first power member can drive the first driving assembly to move, and the first power member is a motor.

7. The test device according to claim 2, further comprising a plugging element, wherein the plugging element is mounted in one of the accommodating channels, and the transmission member can drive the plugging element to move.

8. The test device according to claim 2, wherein the first transmission mechanism further comprises a second power member that provides power to the transmission member, the transmission member is a rack, and the second power member comprises a gear set that meshes with the rack and a motor that drives the gear set.

9. The test device according to claim 1, wherein the test module further comprises a test kit, and the test element is arranged in the test kit.

10. The test device according to claim 9, wherein two ends of a kit body of the test kit are throughly connected to form a through channel, and the test element is connected to the through channel in a sliding and sealing manner, such that the test element can extend out of the through channel or retract into the through channel.

11. The test device according to claim 1, wherein the test element comprises a support frame and test paper laid on the support frame.

12. A test device, comprising:
a container body, the container body having an accommodating channel; and
a test module, the test module being arranged in the accommodating channel, and the test module being movable between a test position and a storage position;
wherein the accommodating channel is provided with a limit member, and the limit member is movable between a limiting position and a yielding position; when the limit member moves to the limiting position, the limit member is in limiting fit with the test module, and the test module is limited to the storage position; when the limit member moves to the yielding position, the limit member is in yielding fit with the test module, and the test module can move between the test position and the storage position.

13. The test device according to claim 12, further comprising a locking member, wherein the locking member is movably arranged on the container body; when the locking member moves to a locking position, the locking member limits the limit member to the limiting position; when the locking member moves to an unlocking position, the locking member releases the limiting of the limit member.

14. The test device according to claim 13, further comprising a base, wherein the container body is detachably mounted on the base; when the container body is assembled in place with the base, the locking member moves from the locking position to the unlocking position under the action of the base.

15. The test device according to claim 14, further comprising an elastic member, wherein the locking member can move to the locking position under the elastic force provided by the elastic member.

16. The test device according to claim 15, wherein the limit member is an anti-falling rib arranged on a cavity wall of the accommodating channel, and the test module is correspondingly provided with a groove in limiting fit with the anti-falling rib; the anti-falling rib has a deformation quantity of movement between the limiting position and the yielding position.

17. The test device according to claim 16, wherein the locking member is provided with a convex portion; when the locking member moves to the locking position, the convex portion abuts against the limit member to limit the movement of the limit member towards the yielding position; when the locking member moves to the unlocking position, the convex portion separates from the limit member to release the limiting of the limit member.

18. The test device according to claim 16, wherein the container body further has a central cavity, the central cavity further comprises a sleeve with an insertion hole, and the insertion hole is used for accommodating a driving shaft on the base; the locking member is sleeved on an outer wall of the sleeve and moves along an axis of the sleeve; a step is provided on the peripheral side of the end of the driving shaft away from the container body, and the step is adapted to the end of the locking member away from the convex portion.

19. The test device according to claim 18, wherein the elastic member is located in a gap between the sleeve and the locking member; the locking member is provided with a first abutting portion, the outer wall of the sleeve is provided with a second abutting portion, and two ends of the elastic member abut against the first abutting portion and the second abutting portion.

20. The test device according to claim 19, wherein the locking member is further provided with a hook, a hanging groove is formed axially on the cavity wall of the central cavity, and the hook is clamped in the hanging groove and is movable along the hanging groove.

21. The test device according to any one of claims 2-3, 6-8, and 11, further comprising:
a base having an accommodating chamber, wherein the container body is movably arranged in the accommodating chamber;
wherein the transmission member is provided with a clamping portion; one end of the test module is provided with a connecting portion;
when the container body drives the test module to move to a first position, the clamping portion separates from the connecting portion, such that the transmission member separates from the test module; when the container body drives the test module to move to a second position, the connecting portion is in limiting fit with the clamping portion, such that the transmission member is in transmission fit with the test module.

22. The test device according to claim 21, wherein the container body is rotatably arranged on the base, and the container body rotates within a preset angle to drive the test module to move between the first position and the second position.

23. The test device according to claim 21, wherein the container body is arranged on the base in a sliding manner, and the container body slides within a preset distance to drive the test module to move between the first position and the second position.

24. The test device according to claim 21, wherein the end, provided with the connecting portion, of the test module can extend into and exit from the accommodating channel, and the connecting portion is in limiting fit with or separates from the clamping portion outside the accommodating channel; when the transmission member is in transmission fit with the test module, and during the process that the connecting portion is in limiting fit with the clamping portion within the accommodating channel, the connecting portion and the clamping portion cannot be separated under the limiting action of the cavity wall of the accommodating channel or the limit ribs in the accommodating channel.

25. The test device according to claim 21, wherein one of the connecting portion and the clamping portion is a buckle, and the other of the connecting portion and the clamping portion is a hook; the buckle is a concave portion with a side opening, and the hook is inserted into the buckle from the side opening.

26. The test device according to claim 21, further comprising a plugging element for plugging a test hole at a bottom of a toilet bowl.

27. A test device, comprising:
a bracket provided with a base; and
a test assembly mounted in the base, wherein the test assembly comprises a container body and a rear cover, a plurality of test modules are mounted in the container body, and the rear cover is movable between a locking position and an unlocking position; when the rear cover moves to the locking position, the rear cover limits the container body in the base; when the rear cover moves to the unlocking position, the container body can exit from the base.

28. The test device according to claim 27, wherein the rear cover is movably mounted on the container body; when the rear cover moves to the locking position, the rear cover is in limiting fit with the base to limit the container body in the base; when the rear cover moves to the unlocking position, the rear cover is in yielding fit with the base, such that the container body can exit from the base; or
the rear cover is movably mounted on the base; when the rear cover moves to the locking position, the rear cover is in limiting fit with the container body to limit the container body in the base; when the rear cover moves to the unlocking position, the rear cover is in yielding fit with the container body, such that the container body can exit from the base.

29. The test device according to claim 27, wherein the container body is provided with a first connecting portion, the rear cover is provided with a second connecting portion, and the first connecting portion is in axial limiting fit and circumferential yielding fit with the second connecting portion along the container body.

30. The test device according to claim 29, wherein one of the first connecting portion and the second connecting portion is a pivot hole, the other of the first connecting portion and the second connecting portion is a pivot shaft, and the pivot shaft is provided with an inverted buckle in clamping fit with the pivot hole.

31. The test device according to claim 27, wherein the base comprises an accommodating chamber, the test assembly is mounted in the accommodating chamber, a front end of the accommodating chamber is provided with a front opening that allows the test module to extend out, a rear end of the accommodating chamber is provided with a rear opening and a limit portion, the rear cover is arranged on the side of the accommodating chamber near the rear opening, and the rear cover is provided with a fit portion; when the rear cover moves to the locking position, the fit portion is in limiting fit with the limit portion, and then the container body is limited in the accommodating chamber by the rear cover; when the rear cover moves to the unlocking position, the fit portion is in yielding fit with the limit portion, and then both the rear cover and the container body can exit the accommodating chamber from the rear opening.

32. The test device according to claim 27, further comprising a first transmission mechanism, wherein the first transmission mechanism comprises a transmission member in transmission fit with the test module, and the rear cover further has a yielding channel; when the rear cover moves to the locking position, the transmission member can extend into the yielding channel to be in transmission fit with the test element.

33. The test device according to claim 27, wherein the container body is rotatably arranged in the base and provided with an insertion hole, the test device further comprises a driving mechanism, the driving mechanism comprises a driving shaft in transmission fit with the container body, the driving shaft is in circumferential limiting fit with the insertion hole to drive the container body to rotate, and the driving mechanism further comprises a motor that drives the driving shaft to rotate.

34. The test device according to claim 27, wherein the container body has a plurality of mutually independent accommodating channels inside, and the plurality of test modules are mounted in corresponding accommodating channels respectively.

35. A test device, comprising:
a bracket having a base;
a test assembly detachably mounted in the base;
a driving mechanism used for driving the test assembly to move;
a positioning member movably arranged on the bracket, wherein when the positioning member moves to a third position, the positioning member can be in limiting fit with the test assembly; when the positioning member moves to a fourth position, the positioning member can be in yielding fit with the test assembly;
a second transmission mechanism used for driving the positioning member to move; and
a main control board electrically connected to the driving mechanism and the second transmission mechanism respectively to control the operation of the driving mechanism and the second transmission mechanism.

36. The test device according to claim 35, wherein the test assembly is mounted in or exits from the base in a direction parallel to its central axis, and the positioning member moves in a direction perpendicular to the central axis of the test assembly.

37. The test device according to claim 35, wherein the test assembly is provided with a third abutting portion; when the positioning member moves to the third position, the positioning member is in limiting fit with the third abutting portion to limit the test assembly along an axis of the test assembly.

38. The test device according to claim 35, wherein the test assembly is provided with a fourth abutting portion; when the positioning member moves to the third position, the positioning member is in limiting fit with the fourth abutting portion to limit the test assembly along a circumference of the base.

39. The test device according to claim 35, wherein the base is provided with a test channel and an accommodating chamber, the test assembly is mounted in the accommodating chamber, the test assembly comprises a container body with a plurality of accommodating channels, the container body switches to move along the accommodating chamber to ensure that each accommodating channel corresponds to the test channel respectively, and the driving mechanism is in transmission fit with the container body to drive the container body to move in the switching direction.

40. The test device according to claim 39, wherein a plurality of positioning grooves are circumferentially arranged at intervals on the periphery of the container body, and the positioning member forms an axial limiting fit with front walls of the positioning grooves to limit the test assembly from exiting the accommodating chamber; the positioning member forms a circumferential limiting fit with side walls of the positioning grooves to position the movement of the container body.

41. The test device according to claim 35, wherein the second transmission mechanism comprises a driving motor and a second gear set arranged at an output end of the driving motor, the positioning member is a baffle, and one side of the baffle is provided with teeth matching the second gear set.

42. A toilet, comprising the test device according to any one of claims 1-11 and 21-26, wherein a toilet bowl of the toilet is provided with a test hole into which the test element extends to test a liquid under test.

43. The toilet according to claim 42, wherein:
the test device further comprises a driving mechanism and a plugging element, wherein the driving mechanism is in transmission fit with the container body to drive the container body to move; the plugging element is provided in one of the accommodating channels in an extensible and retractable manner, and the plugging element after extending out can be in sealing fit with the test hole; and
the position of the test hole corresponds to the test module or the plugging element; when test is performed, the driving mechanism drives the container body to move, such that the test module corresponds to the test hole; when no test is performed, the driving mechanism drives the container body to move, such that the plugging element corresponds to the test hole.

44. The toilet according to claim 43, further comprising a first transmission mechanism, wherein when the test module corresponds to the test hole, the first transmission mechanism drives the test element to extend out from the test hole for test; when the plugging element corresponds to the test hole, the first transmission mechanism drives the plugging element to extend out to plug the test hole.

45. A test method, comprising the following process: providing a container body used for storing test modules, wherein the container body is provided with a plurality of mutually independent accommodating channels inside, the test module is mounted in the accommodating channel, and the test module is provided with a test element for test a liquid under test; a first transmission mechanism drives the test module to extend out of the corresponding accommodating channel; the first transmission mechanism moves or the container body moves, and wherein the first transmission mechanism can drive the test module to extend out of the corresponding accommodating channel.

46. The test method according to claim 45, comprising the following process:
when test is performed, the container body moves to a position where one test module corresponds to a test hole of a toilet, and the first transmission mechanism drives the test module to extend out of the corresponding accommodating channel; and
after the test is completed, the test module retracts into the original accommodating channel; or discarding the test module.
